# EUROPEAN PATENT APPLICATION

(11) **EP 3 756 600 A1**
(43) Date of publication of application: **30.12.2020**
(21) Application number: 20181858.0
(22) Date of filing: 24.06.2020
(51) Int. Cl.: A61B 18/14

(54) **IRRIGATION FLUID MONITOR AND ALARM**

(30) Priority: 25.06.2019 US 201962866106 P; 19.05.2020 US 202016877711
(71) Applicant: Biosense Webster (Israel) Ltd, Yokneam, 2066717 (IL)
(72) Inventor: KELLY, Christopher P., Irwindale, CA 91706 (US); GONZALEZ, Daniel, Irwindale, CA 91706 (US); KASTIN, Vadim, Irwindale, CA 91706 (US)
(74) Representative: Small, Gary James

(57) **Abstract**

An apparatus includes a fluid bag support coupling, a signal generating assembly, and a signal output driver circuit. The fluid bag support coupling is configured to support a fluid source. The signal generating assembly is configured to generate a signal in response to an amount of fluid within the fluid source. The signal output driver circuit is in electrical communication with the signal generating assembly. The signal output driver circuit is configured to transmit the signal from the signal generating assembly to a working element configured to perform a task. The signal is configured to inhibit the working element from performing the task when the amount of fluid within the fluid source reaches a predetermined threshold amount.

## Description

### PRIORITY

This application claims priority to U.S. Provisional Pat. App. No. 62/866,106, entitled "Irrigation Fluid Monitor and Alarm," filed June 25, 2019, the disclosure of which is incorporated by reference herein in its entirety.

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad or other reference electrode that is in contact with the patient that is in contact with the patient. Irrigation may be used to draw heat from ablating components of an ablation catheter; and to prevent the formation of blood clots near the ablation site.

Examples of ablation catheters are described in U.S. Pub. No. 2013/0030426, entitled "Integrated Ablation System using Catheter with Multiple Irrigation Lumens," published January 31, 2013, the disclosure of which is incorporated by reference herein in its entirety; U.S. Pub. No. 2017/0312022, entitled "Irrigated Balloon Catheter with Flexible Circuit Electrode Assembly," published November 2, 2017, the disclosure of which is incorporated by reference herein in its entirety; U.S. Pub. No. 2018/0071017, entitled "Ablation Catheter with a Flexible Printed Circuit Board," published March 15, 2018, the disclosure of which is incorporated by reference herein in its entirety; U.S. Pub. No. 2018/0056038, entitled "Catheter with Bipole Electrode Spacer and Related Methods," published March 1, 2018, the disclosure of which is incorporated by reference herein in its entirety; U.S. Pat. No. 10,130,422, entitled "Catheter with Soft Distal Tip for Mapping and Ablating Tubular Region," issued November 20, 2018, the disclosure of which is incorporated by reference herein in its entirety; U.S. Pat. No. 8,956,353, entitled "Electrode Irrigation Using Micro-Jets," issued February 17, 2015, the disclosure of which is incorporated by reference herein in its entirety; and U.S. Pat. No. 9,801,585, entitled "Electrocardiogram Noise Reduction," issued October 31, 2017, the disclosure of which is incorporated by reference herein in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein in its entirety; U.S. Pat. No. 10,130,422, entitled "Catheter with Soft Distal Tip for Mapping and Ablating Tubular Region," issued November 20, 2018, the disclosure of which is incorporated by reference herein in its entirety; and U.S. Pub. No. 2018/0056038, entitled "Catheter with Bipole Electrode Spacer and Related Methods," published March 1, 2018, the disclosure of which is incorporated by reference herein in its entirety.

When using an ablation catheter, it may be desirable to ensure that the one or more electrodes of the ablation catheter are sufficiently contacting target tissue. For instance, it may be desirable to ensure that the one or more electrodes are contacting target tissue with enough force to effectively apply RF ablation energy to the tissue; while not applying a degree of force that might tend to undesirably damage the tissue. To that end, it may be desirable to include one or more force sensors or pressure sensors to detect sufficient contact between one or more electrodes of an ablation catheter and target tissue.

In addition to using force sensing or EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3® system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein in its entirety; and various other references that are cited herein.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of a distal portion of the catheter of FIG. 1, with additional components shown in schematic form;
FIG. 3 depicts a perspective view of the distal portion of the catheter of FIG. 1, with an outer sheath omitted to reveal internal components;
FIG. 4 depicts an exploded perspective view of the distal portion of the catheter of FIG. 1;
FIG. 5 depicts a schematic view of another medical procedure in which a catheter assembly is inserted in a patient, where an irrigation fluid monitor and alarm is coupled to a fluid source;
FIG. 6A depicts a front elevation view of the irrigation fluid monitor and alarm of FIG. 5;
FIG. 6B depicts a front elevation view of the irrigation fluid monitor and alarm of FIG. 5 coupled with a fluid bag full of irrigation fluid;
FIG. 6C depicts a front elevation view of the irrigation fluid monitor and alarm of FIG. 5 coupled with a fluid bag empty of irrigation fluid; and
FIG. 7 depicts a schematic view of the irrigation fluid monitor and alarm of FIG. 5.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Exemplary Ablation Catheter System

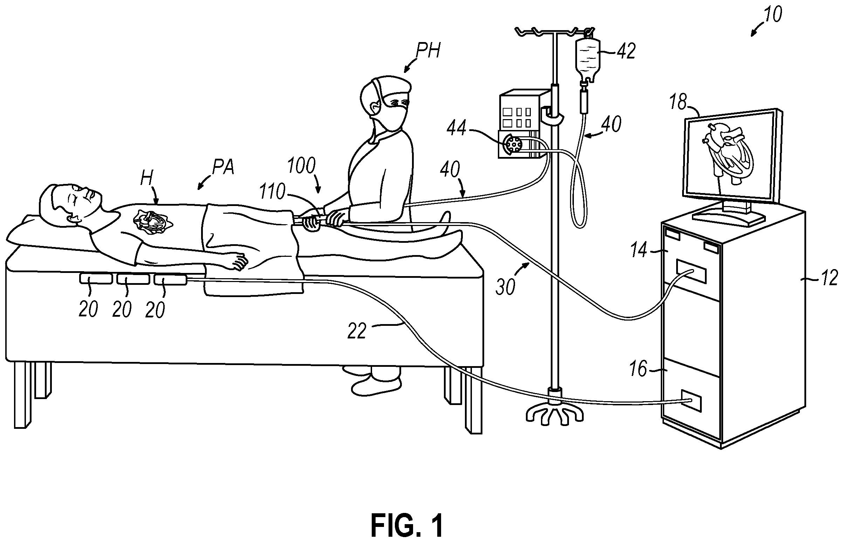

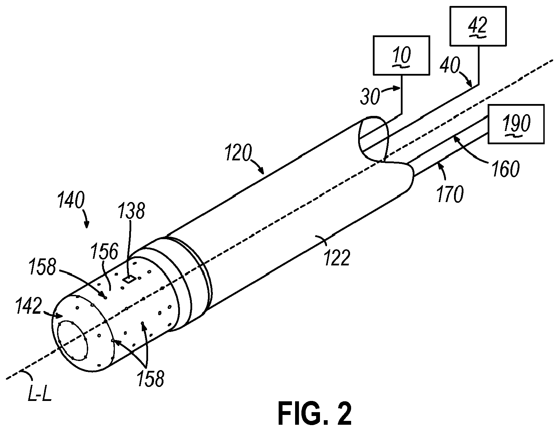

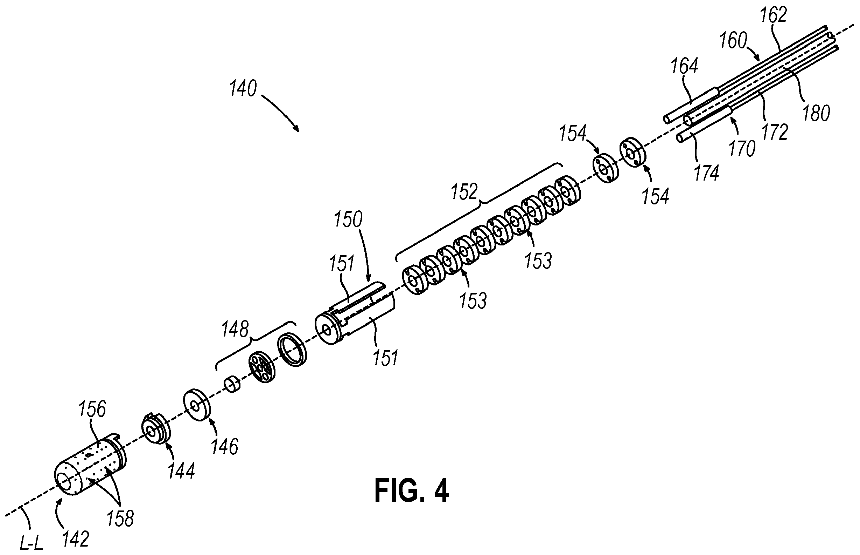

FIG. 1 shows an exemplary medical procedure and associated components of a cardiac ablation catheter system that may be used to provide cardiac ablation as referred to above. In particular, FIG. 1 shows a physician (PH) grasping a handle (110) of a catheter assembly (100), with an end effector (140) of a catheter (120) (shown in FIGS. 2 and 4 but not shown in FIG. 1) of catheter assembly (100) disposed in a patient (PA) to ablate tissue in or near the heart (H) of the patient (PA). Catheter assembly (100) includes handle (110), catheter (120) extending distally from handle (110), end effector (140) located at a distal end of catheter (120), and a user input feature (190) located on handle.

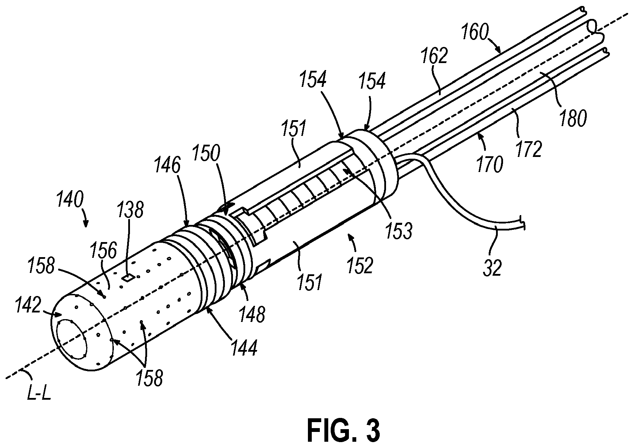

As will be described in greater detail below, end effector (140) includes various components configured to deliver RF energy to targeted tissue sites, provide EP mapping functionality, track external forces imparted on end effector (140), track the location of end effector (140), and disperse irrigation fluid. As will also be described in greater detail below, user input feature (190) is configured to deflect end effector (140) and a distal portion of catheter (120) away from a central longitudinal axis (L-L) (FIGS. 3-5) defined by a proximal portion of catheter (120).

As shown in FIG. 2, catheter (120) includes an elongate flexible sheath (122), with end effector (140) being disposed at a distal end of sheath (122). End effector (140) and various components that are contained in sheath (122) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Field generators (20) are merely optional.

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via microelectrodes (138) of end effector (140) as described in greater detail below. Console (12) includes a processor (not shown) that processes such EP mapping signals and thereby provides EP mapping as is known in the art.

First driver module (14) of the present example is further operable to provide RF power to a distal tip member (142) of end effector (140), as will be described in greater detail below, to thereby ablate tissue. Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly (150) in end effector (140). In such versions, the processor of console (12) is also operable to process the position indicative signals from navigation sensor assembly (150) to thereby determine the position of end effector (140) within the patient (PA). As will be described in greater detail below, navigation sensor assembly (150) includes a pair of coils on respective panels (151) that are operable to generate signals that are indicative of the position and orientation of end effector (140) within the patient (PA). The coils are configured to generate electrical signals in response to the presence of an alternating electromagnetic field generated by field generators (20). Other components and techniques that may be used to generate real-time position data associated with end effector (140) may include wireless triangulation, acoustic tracking, optical tracking, inertial tracking, and the like. Alternatively, end effector (140) may lack a navigation sensor assembly (150).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from navigation sensor assembly (150) of end effector (140). For instance, as end effector (140) of catheter (120) moves within the patient (PA), the corresponding position data from navigation sensor assembly (150) may cause the processor of console (12) to update the patient anatomy views in display (18) in real time to depict the regions of patient anatomy around end effector (140) as end effector (140) moves within the patient (PA). Moreover, the processor of console (12) may drive display (18) to show locations of aberrant conductive tissue sites, as detected via electrophysiological (EP) mapping with end effector (140) or as otherwise detected (e.g., using a dedicated EP mapping catheter, etc.). The processor of console (12) may also drive display (18) to superimpose the current location of end effector (140) on the images of the patient's anatomy, such as by superimposing an illuminated dot, a crosshair, a graphical representation of end effector (140), or some other form of visual indication.

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). As described in greater detail below, such irrigation fluid may be expelled through openings (158) of distal tip member (142) of end effector (140). Such irrigation may be provided in any suitable fashion as will be apparent to those skilled in the art in view of the teachings herein.

### II. Exemplary End Effector of Catheter Assembly

FIGS. 2-4 show exemplary components of end effector (140), and other components of the distal portion of catheter (120), in greater detail. End effector (140) includes a distal tip member (142), a distal tip base (144), a distal circuit disk (146), a strain gauge assembly (148), a navigation sensor assembly (150), a distal spacer stack (152), and a pair of proximal spacers (154). Distal tip member (142), distal tip base (144), distal circuit disk (146), strain gauge assembly (148), navigation sensor assembly (150), distal spacer stack (152), and proximal spacers (154) are coaxially aligned with each other and are stacked longitudinally so that these components (144-154) define a stacked circuit. A pair of push-pull cables (160, 170) and an irrigation tube (180) extend along the length of catheter (120) to reach end effector (140). Each of the foregoing components will be described in greater detail below. Flexible sheath (122) surrounds all of the foregoing components except for distal tip member (142).

As shown in FIGS. 3-4, distal tip member (142) of the present example is electrically conductive and includes a cylindraceous body (156) with a dome tip. A plurality of openings (158) are formed through cylindraceous body (156) and are in communication with the hollow interior of distal tip member (142). Openings (158) thus allow irrigation fluid to be communicated from the interior of distal tip member (142) out through cylindraceous body (156). Cylindraceous body (156) and the dome tip are also operable to apply RF electrical energy to tissue to thereby ablate the tissue. Such RF electrical energy may be communicated from first driver module (14) to the proximal-most spacer (154) via cable (30). Distal tip member (142) may also include one or more thermocouples that are configured to provide temperature sensing capabilities.

As shown in FIGS. 3-4, distal tip member (142) of the present example also includes one or more EP mapping microelectrodes (138) mounted to cylindraceous body (156). EP mapping microelectrodes (138) are configured to pick up electrical potentials from tissue that comes into contact with EP mapping microelectrodes (138). First driver module (14) may process the EP mapping signals and provide the physician (PH) with corresponding feedback indicating the locations of aberrant electrical activity in accordance with the teachings of various references cited herein.

Strain gauge assembly (148) is positioned proximal to distal circuit disk (146) and is configured to sense external forces that impinge against distal tip member (142). When distal tip (142) encounters external forces (e.g., when distal tip (142) is pressed against tissue), those external forces are communicated from distal tip (142) to distal tip base (144), to distal circuit disk (146), and to strain gauge assembly (148) such that strain gauge may generate a suitable signal corresponding to the magnitude and direction of the external force.

Navigation sensor assembly (150) may generate signals indicating the position and orientation of end effector (140) in three-dimensional space with substantial precision. The signals from navigation sensor assembly (150) may be communicated through vias or other structures in the layers that are proximal to strain navigation sensor assembly (150), eventually reaching first driver module (14) of console (12) via cable (30).

As noted above and as shown in FIGS. 1-2, cable (30) couples catheter assembly (100) with drive system (10). As shown in FIG. 4, wires (32) of cable (30) extend along the length of catheter (120) to reach the proximal-most proximal spacer (154).

As also noted above, catheter assembly (100) is configured to enable irrigation fluid to be communicated from fluid source (42) to catheter (120) via fluid conduit (40), thereby providing expulsion of the irrigation fluid via openings (158) of distal tip member (142). In the present example, the fluid path for the irrigation fluid includes an irrigation tube (180), which is shown in FIGS. 3-4. The proximal end of irrigation tube (180) is coupled with fluid conduit (40) (e.g., at handle (110) of catheter assembly (100)). Irrigation tube (180) extends along the length of catheter (120) to reach end effector (140). In some versions, irrigation fluid may be communicated from the distal end of irrigation tube (180) through the central passageway formed by the aligned by the above-mentioned central apertures, ultimately reaching the interior of distal tip member (142) via aperture (158) of distal tip base (144).

As noted above, and as shown in FIGS. 2-4, catheter (100) of the present example further includes a pair of push-pull cables (160, 170). Push-pull cables (160, 170) enable the physician (PH) to selectively deflect end effector (140) laterally away from a longitudinal axis (L-L), thereby enabling the physician (PH) to actively steer end effector (140) within the patient (PA). Various mechanisms that may be used to drive push-pull cables (160, 170) in a simultaneous, longitudinally-opposing fashion will be apparent to those skilled in the art in view of the teachings herein.

### III. Exemplary Irrigation Fluid Monitor and Alarm

As mentioned above, distal tip (142) of end effector (140) defines a plurality of openings (158) configured to allow irrigation fluid to be communicated from the interior to the exterior of distal tip member (142). As also mentioned above, end effector (140) is configured to be disposed within or near the heart (H) of the patient (PA) to ablate tissue. Therefore, during exemplary use, pump (44) may drive irrigation fluid from fluid source (42), through conduit (40) and irrigation tube (180), and into the interior of distal tip member (142) such that irrigation fluid originating from fluid source (42) may flow out of the plurality of openings (158) into or near the heart (H) of the patient (PA).

If irrigation fluid is sufficiently depleted from fluid source (42) during use of catheter assembly (100) in accordance with the description above, pump (44) may attempt to drive air from empty fluid source (42), rather than the intended irrigation fluid. If air is pumped through conduit (40), irrigation tube (180), and out of the plurality of openings (158) at distal tip (142) of end effector (140) while end effector (140) is deposed in or near the heart (H) of the patient (PA), the pumped air may cause undesirable consequences, such as an air embolism. Therefore, it may be desirable to automatically shut off pump (44) when fluid source (42) either is sufficiently empty of irrigation fluid or is just about to become sufficiently empty of irrigation fluid.

Irrigation fluid may be used during exemplary use of catheter assembly (100) to sufficiently cool end effector (140) or surrounding tissue in response to activating end effector (140) with RF energy in accordance with the description above. If irrigation fluid is sufficiently depleted from fluid source (42) during use, activating end effector (140) with RF energy may cause undesirable consequences, such as excessive tissue ablation or excessive thermal spreading to adjacent tissue. Therefore, it may be desirable to automatically inhibit end effector (140) from activating RF energy when fluid source (42) either is sufficiently empty of irrigation fluid or is just about to become sufficiently empty of irrigation fluid.

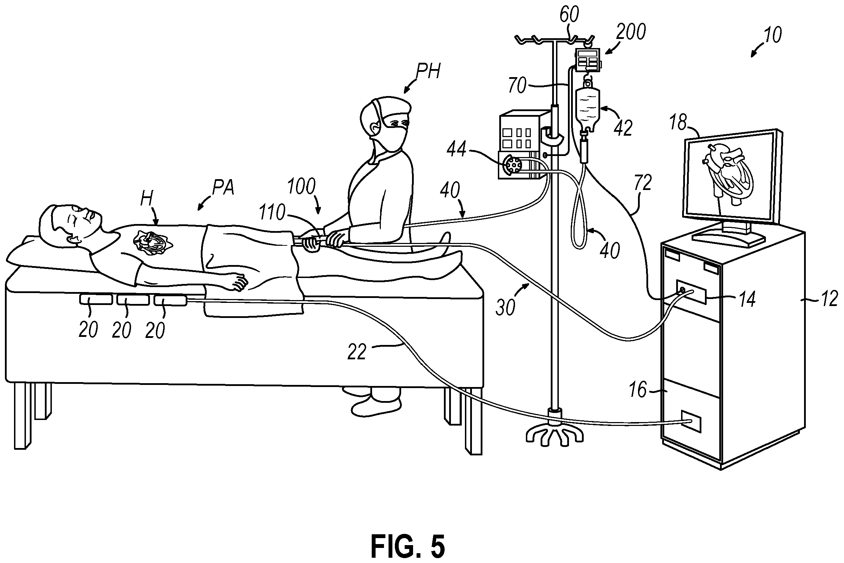

FIG. 5 shows the physician (PH) utilizing catheter assembly (100) in conjunction with guidance and drive system (10), field generators (20), fluid source (42), and pump (44), similar to that shown in FIG. 1, except with the additional use of an exemplary irrigation fluid monitor (200). In particular, during exemplary use, fluid monitor (200) is coupled to fluid source (42) such that fluid monitor (200) supports the weight of fluid source (42). As will be described in greater detail below, fluid monitor (200) is configured to monitor the amount of irrigation fluid housed within fluid source (42). As will also be described in greater detail below, once fluid monitor (200) detects an amount of irrigation fluid within fluid source (42) below a predetermined threshold volume/weight, fluid monitor (200) is configured to shut off pump (44), deactivate RF energy supplied to end effector (140), generate an audible alarm, or any suitable combination of the above actions.

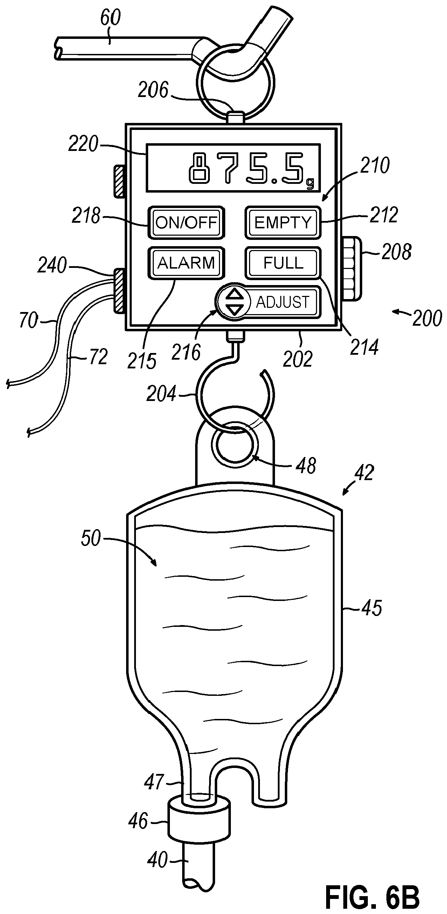

As best seen in FIG. 6B, fluid source (42) includes a fluid bag (45) initially filled with irrigation fluid (50). The lower portion of fluid bag (45) includes a fluid exit (47) configured to fluidly couple the interior of fluid bag (45) with fluid conduit (40) via a coupler (46). Therefore, irrigation fluid (50) may travel from the interior of fluid bag (45) into fluid conduit (40) via coupler (46) and fluid exit (47).

The upper portion of fluid bag (45) defines a top opening (48) that is configured to receive a fluid bag coupling hook (204) of fluid monitor (200) such that coupling hook (204) supports the weight of fluid bag (45). While hook (204) is used to couple fluid bag (25) with fluid monitor (200) in the current example, any other suitable coupling body may be used as would be apparent to one skilled in the art in view of the teachings herein. Top opening (48) may be fluidly isolated from irrigation fluid (50) housed within fluid bag (45).

In the current example, fluid monitor (200) is supported by an IV pole (60) via an upper coupler hook (206). However, upper coupler hook (206) may have any other suitable coupling body as would be apparent to one skilled in the art in view of the teachings herein. Additionally, fluid monitor (200) may be attached to any suitable structure as would be apparent to one of skill in the art in view of the teachings herein. In some instances, fluid monitor (200) may be a self-standing structure.

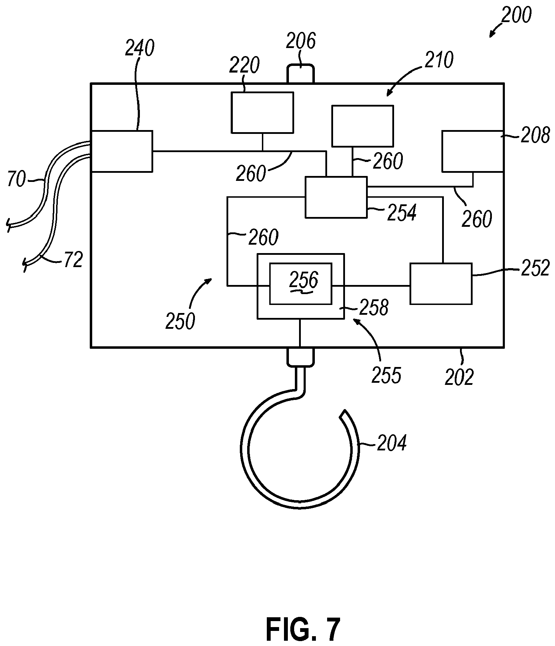

As best seen in FIG. 7, fluid monitor (200) includes a casing (202), fluid bag coupling hook (204), upper coupling hook (206), an audible alarm (208), a user input assembly (210), a digital display (220), a signal output driver circuit (240), and a signal generating loop assembly (250). Casing (202) accommodates coupling hooks (204, 206), audible alarm (208), user input assembly (210), digital display (220), signal output driver circuit (240), and signal generating loop assembly (250), such that fluid monitor (200) may travel as a single unit. In some versions, signal output driver circuit (240) may include a load cell analog circuit providing a ranging from approximately 4 mA to approximately 20 mA. For instance, when zero mass is present, signal output driver circuit (240) outputs 4mA; and when at full load (e.g., as determined by the user interface provided to the physician or nurse), signal output driver circuit (240) outputs a 20mA signal.

Auditable alarm (208) is in electrical communication with a receiver (254) of signal generating loop assembly (250). Audible alarm (208) is configured to generate noise when activated. As will be described in greater detail below, receiver (254) is configured to activate audible alarm (208), thereby notifying the physician (PH), when receiver (254) measures an electrical current within signal generating loop assembly (250) indicative of fluid source (42) housing an amount of irrigation fluid (50) below a predetermined threshold volume/weight.

Digital display (220) is in electrical communication with receiver (254) of signal generating loop assembly (250). Digital display (220) is configured to display a value indicative of the amount of irrigation fluid (50) within fluid source (42) based on an electrical current within signal generating loop assembly (250) measured by receiver (254). Digital display (220) may display the value indicative of the amount of irrigation fluid (50) within fluid source as any suitable unit as would be apparent to one skilled in the art in view of the teachings herein. For instance, digital display (200) may be configured to display the value in grams, milliliters, etc.

User input assembly (210) is also in electrical communication with receiver (254) or signal generating loop assembly (250). User input assembly (210) includes a zeroing button (212), a spanning button (214), an adjustment controls (216), a power button (218), and an alarm button (215). Power button (218) is configured to activate and deactivate irrigation fluid monitor (200). Alarm button (215) may be pressed to deactivate audible alarm (208).

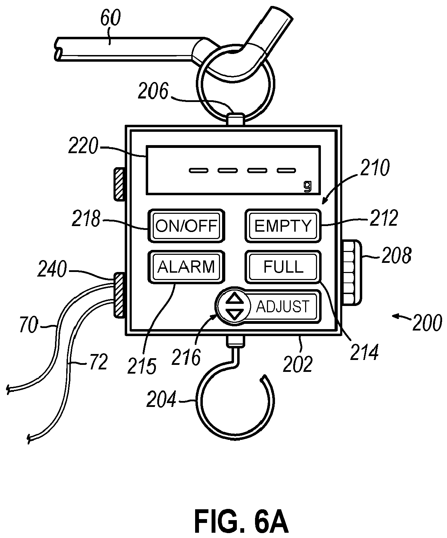

Zeroing button (212), spanning button (214), and adjusting button assembly (216) are configured to calibrate receiver (254) such that the signal measured by receiver (254) is proportionate to the amount of irrigation fluid (50) within fluid source (42). For example, as shown in FIG. 6A, prior to using fluid source (42) with pump (44) in accordance with the description herein, a physician (PH) may press zeroing button (212), thereby communicating to receiver (254) the weight measured on hook (204) at that moment correlates to an unweighted hook (204). Next, as shown in FIG. 6B, The physician (PH) may also couple a full fluid bag (45) to hook (204) and then press the span button (214), thereby communicating to receiver (254) the weight measured on hook (204) at that moment correlates to a full fluid bag (45). If the physician (PH) happens to have a fluid bag (45) that is partially full, they may use adjustment controls (216) to input how much fluid (50) is in bag (45), thereby communicating to receiver (254) the weight measured on hook (204) at that moment.

Signal output driver circuit (240) is in electrical communication with receiver (254). Signal output driver circuit (240) is coupled with pump communication wire (70) and first driver module communication wire (72). Pump communication wire (70) electrically couples with pump (44) such that receiver (254) may send signals to pump (44) via signal output driver circuit (240) and wire (70). Similarly, first driver module communication wire (72) electrically couples with first driver module (14) such that receiver (254) may send signals to first driver module (14) via signal output driver circuit (240) and wire (72).

Receiver (254) may be in bidirectional communication with both pump (44) and first driver module (14) such that pump (44) and first driver module (14) may also send information to receiver (254). Signal output driver circuit (240) may selectively couple with pump communication wire (70) and first driver module communication wire (72). Alternatively, signal output driver circuit (240) may be permanently attached to pump communication wire and first driver module communication wire (72).

As will be described in greater detail below, receiver (254) may send signals to pump (44) and first driver module (14) in order to deactivate pump (44) and inhibit first driver module (14) from activating end effector (140) with RF energy when receiver (254) measures an electrical signal within signal generating loop assembly (250) indicative of fluid source (42) housing an amount of irrigation fluid (50) below a predetermined threshold volume/weight.

Signal generating loop assembly (250) includes a power source (252), a receiver (254), and a load cell assembly (255). As will be described in greater detail below, signal generating loop assembly (250) is configured to generate an electrical signal that is indicative of the amount of irrigation fluid (50) housed within fluid bag (45). Additionally, as will be described in greater detail below, signal generating loop assembly (250) is configured to communicate the electrical signal indicative of the amount of irrigation fluid (50) housed within fluid bag (45) to audible alarm (208), digital display (220), and signal output driver circuit (240).

Power source (252) is configured to electrically power other suitable components of fluid monitor (200). Power source (252) may include any suitable type of battery as would be apparent to one skilled in the art in view of the teachings herein. Additionally or alternatively, power source (252) may be configured to couple with an outside supply of power, such as console (12), a generator, a wall outlet, etc. In some instances, power source (252) may be configured to couple with an outside power source in order to charge batteries of power source (252). Power source (252) may be in direct communication with audible alarm (208), digital display (220), user input assembly (210), etc. Alternatively, there may be a first power source (252) within signal generating loop assembly (250), and a second power source that electrically powers all other components of irrigation fluid monitor (200).

Power source (252) is electrically coupled with receiver (254) and load cell assembly (255) via electrical coupling (260) in order to form signal generating loop assembly (250). During exemplary use, power source (252) may generate sufficient power to create an electrical signal traveling through signal generating loop assembly (250). As will be described in greater detail below, load cell assembly (255) is configured to modify the electrical signal traveling through signal generating loop assembly (250) in response to the load supported by hook (204).

Receiver (254) is configured to suitably process the electrical signal within signal generating loop assembly (250) and communicate proportional signals to audible alarm (208), digital display (220), and signal output driver circuit (240) in accordance with the description herein. Receiver (254) may include any suitable components as would be apparent to one skilled in the art in view of the teachings herein. For instance, receiver (254) may be configured in accordance with signal generating loop assembly (250) providing a 4-20 mA direct current loop. Therefore, when load cell assembly (255) experiences a load supported by hook (204) associated with a fluid source (42) that is full of irrigation fluid (50), signal generating loop assembly (250) may produce a 20 mA electrical current measured by receiver (254); while when load cell assembly (255) experiences a load supported by hook (204) associated with fluid source (42) having an amount of irrigation fluid (50) below a predetermined threshold volume/weight, signal generating loop assembly (250) may produce a 4 mA electrical current measured by receiver (254).

Load cell assembly (255) includes strain gauge assembly (256) and elastic mechanical ground body (258). Strain gauge assembly (256) may include any suitable component(s) as would be apparent to one skilled in the art in view of the teachings herein. Similar, elastic mechanical ground body (258) may include any suitable material as would be apparent to one skilled in the art in view of the teachings herein, such as any suitable metal, alloy, etc.

Strain gauge assembly (256) is fixed to elastic mechanical ground body (258), while elastic mechanical ground body (258) is fixed to casing (202). Fluid coupling hook (204) is coupled with elastic mechanical ground body (258) such that loads supported by fluid coupling hook (204) are in turn supported by elastic mechanical ground body (258). In particular, loads transferred from fluid coupling hook (204) to elastic mechanical ground body (258) may proportionally cause elastic mechanical ground body (258) to elastically deform. Therefore, when fluid coupling hook (204) supports fluid source (42), the weight of fluid source (42) may cause elastic mechanical ground body (258) to elastically deform in proportion to the amount of irrigation fluid (50) housed within fluid source (50). Therefore, as irrigation fluid (50) is depleted from fluid source (42) in accordance with the description herein, the elastic deformation of mechanical ground body (258) will change proportionately. In other words, the more irrigation fluid (50) that is within fluid source (42) coupled to hook (204), the greater the elastic deformation of mechanical ground body (258); while the less irrigation fluid (50) that is within fluid source (42) coupled to hook (204), the less the elastic deformation of mechanical ground body (258).

Strain gauge assembly (256) is electrically coupled to both receiver (254) and power source (252) via electrical couplings (260) such that the electrical signal within loop (250) travels through strain gauge assembly (256). Since strain gauge assembly (256) is attached to mechanical ground body (258), strain gauge assembly (256) also elastically deforms in response to mechanical ground body (258) supporting fluid source (42), in accordance with the description above. The elastic deformation of strain gauge assembly (256) changes the resistance of strain gauge assembly (256), which in turn modifies the electrical signal traveling through signal generating loop assembly (250). The change in resistance of strain gauge assembly (256) and the electrical signal traveling through signal generating loop assembly (250) may be proportionate to the elastic deformation of elastic mechanical ground body (258), and therefore may be proportionate to the amount of irrigation fluid (50) within irrigation source (42).

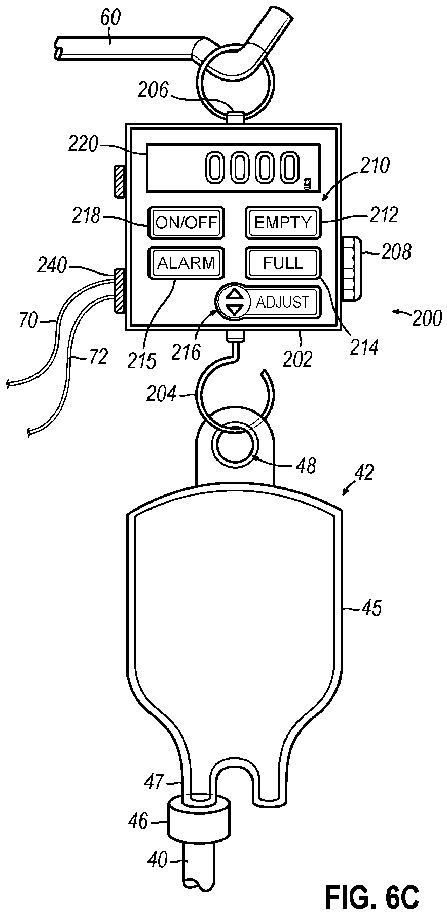

Therefore, signal generating loop assembly (250) may generate a first signal (e.g., electrical current value) measured by receiver (254) when fluid source (42) is full of irrigation fluid (50), as shown in FIG. 6B; while signal generating loop assembly (250) may generate a second signal (e.g., electrical current value) measure by receiver (254) when fluid source (42) is below a predetermined threshold volume/weight of irrigation fluid (50), such as being empty of irrigation fluid (50) as shown in FIG. 6C. Additionally, signal generating loop assembly (250) may generate a proportionate current value measured by receiver (254) when fluid source (42) is between being full and the predetermined threshold volume/weight.

In examples where signal generating loop assembly (250) is a 4-20 mA direct current loop, receiver (254) may measure a 20 mA current when fluid source (42) is full of irrigation fluid (50), while receiver (254) may measure a 4mA current when fluid source (42) is empty of irrigation fluid or below a predetermined threshold volume/weight of irrigation fluid (50).

Due to the proportionate response of current measured within signal generating loop assembly (250) based on the load supported by hook (204) (i.e. the volume/weight of irrigation fluid (50) within fluid source (42)), receiver (254) may be configured to determine a volume/weight within fluid source (42) due to the current measured within signal generating loop assembly (250). While electrical current values are utilized in the present example to identify the load supported by hook (204), other versions may provide different variations within electrical signals (e.g., voltages, resistances, capacitances, inductances, etc.) to represent different loads supported by hook (204). Other suitable electrical signal protocols, and corresponding sensing hardware, that may be used will be apparent to those skilled in the art in view of the teachings herein.

Receiver (254) may communicate the measured current within loop assembly (250) indicative of the amount of irrigation fluid (50) that remains within a fluid bag (45) (or the associated volume/weight within fluid source (42) corresponding to the measured current) to digital display (220) such that the physician may be able to directly see the amount of irrigation fluid (50) that remains within a specific fluid bag (45) during an exemplary procedure. Receiver (254) may also communicate the measured current within loop assembly (250) indicative of the amount of irrigation fluid (50) that remains within a fluid bag (45) (or the associated volume/weight corresponding to the measured current) to first driver module (14), pump (44), audible alarm (208).

Once fluid source (42) is below a predetermined threshold volume/weight of irrigation fluid (50), receiver (254) may send a signal to driver module (14) that atomically inhibits first driver module (14) from activating end effector (140) with RF energy. Therefore, irrigation fluid monitor (200) may inhibit end effector (140) from generating excessive temperatures or excessive tissue ablation.

Additionally, once fluid source (42) is below a predetermined threshold volume/weight of irrigation fluid (50), receiver (254) may send a similar signal to pump (44) that automatically inhibits pump (44) from pumping fluid from fluid source (42) and out of plurality of openings (158) in accordance with the description above. Therefore, irrigation fluid monitor (200) may help prevent pump (44) from inadvertently pumping air through conduit (40), irrigation tube (180), and out of the plurality of openings (158).

Similarly, once fluid source (42) is below a predetermined threshold volume/weight of irrigation fluid (50), receiver (254) may send a signal to audible alarm (208), which may activate in order to alert the physician (PH) to the fact that more irrigation fluid (50) is needed.

In the example where signal generating loop assembly (250) is a 4-20 mA direct current loop, the signal that is configured to deactivate pump (44), inhibit first driver module (14), and activate audible alarm (208) may be a 4 mA electrical current.

While in the current example, load cell assembly (255) includes a strain gauge (256), any suitable measurement device may be used as would be apparent to one skilled in the art in view of the teachings herein.

Irrigation fluid monitor (200) may also include a redundant run dry sensor that may electrically ground the irrigation fluid (50) through irrigation fluid monitor (200). The run dry sensor would be redundant in the fact the sensor may indicate when irrigation fluid (50) within fluid bag (45) is sufficiently empty. By way of example only, such a sensor may include a sterile luer hub with a small chamber containing two small conductive strips (e.g., gold) that are parallel with each other and spaced apart from each other. A circuit would be completed between these two conductive strips when the conductive solution (e.g., 0.9% saline) is in contact with both of the conductive strips simultaneously, thus indicating the presence of irrigation fluid. A small amount of current would be passed from one of the strips. When the current is interrupted, an audible alarm (and/or other response) may be triggered.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus, comprising: (a) a fluid bag support coupling configured to support a fluid source; (b) a signal generating assembly coupled to the fluid bag support coupling, the signal generating assembly being configured to generate a signal in response to an amount of fluid within the fluid source; and (c) a signal output driver circuit in electrical communication with the signal generating assembly, the signal output driver circuit being configured to transmit the signal from the signal generating assembly to a working element configured to perform a task, the signal being configured to inhibit the working element from performing the task when the amount of fluid within the fluid source reaches a predetermined threshold amount.

### Example 2

The apparatus of Example 1, the signal generating assembly comprising a load cell assembly.

### Example 3

The apparatus of any one or more of Examples 1 through 2, the signal generating assembly comprising a power source.

### Example 4

The apparatus of Example 3, the signal generating assembly comprising a receiver in electrical communication with the load cell assembly and the power source.

### Example 5

The apparatus of Example 4, the apparatus comprising a digital display, the receiver being configured to transmit the signal to a digital display.

### Example 6

The apparatus of any one or more of Examples 4 through 5, further comprising an audible alarm, the receiver being configured to activate the audible alarm when the amount of fluid within the fluid source reaches the predetermined threshold amount.

### Example 7

The apparatus of any one or more of Examples 1 through 6, further comprising a user input assembly configured to calibrate the signal generating assembly.

### Example 8

The apparatus of Example 7, the user input assembly further comprising a power button configured to activate the apparatus.

### Example 9

The apparatus of any one or more of Examples 7 through 8, the input assembly further comprising an alarm button configured to deactivate the audible alarm.

### Example 10

The apparatus of any one or more of Examples 1 through 9, the signal generating assembly comprising a run dry sensor.

### Example 11

The apparatus of any one or more of Examples 1 through 10, the working element comprising a pump in fluid communication with the fluid source, the pump being configured to pump fluid out of the fluid source.

### Example 12

The apparatus of any one or more of Examples 1 through 11, the working element comprising a driver module configured to activate RF energy.

### Example 13

The apparatus of any one or more of Examples 1 through 12, the signal generating assembly comprising a strain gauge.

### Example 14

The apparatus of Example 13, the signal generating assembly comprising an elastic body fixed to the strain gauge.

### Example 15

The apparatus of any one or more of Examples 1 through 14, further comprising a body housing at least a portion of the signal generating assembly.

### Example 16

An apparatus, comprising: (a) a fluid bag support coupling configured to support a fluid source; (b) a signal generating assembly coupled to the fluid bag support coupling, the signal generating assembly being configured to generate a signal in response to an amount of fluid within the fluid source; and (c) a signal output driver circuit in electrical communication with the signal generating assembly, the signal output driver circuit being configured to transmit the signal from the signal generating assembly to a pump in communication with the fluid source, the signal being configured to shut off the pump when the amount of fluid within the fluid source reaches a predetermined threshold amount.

### Example 17

The apparatus of Example 16, the signal generating assembly comprising a load cell.

### Example 18

The apparatus of any one or more of Examples 16 through 17, further comprising an audible alarm, the signal generating assembly being configured to activate the audible alarm when the fluid source reaches the predetermined threshold amount.

### Example 19

An apparatus, comprising: (a) a fluid bag support coupling configured to support a fluid source; (b) a signal generating assembly coupled to the fluid bag support coupling, the signal generating assembly being configured to generate a signal in response to an amount of fluid within the fluid source; and (c) a signal output driver circuit in electrical communication with the signal generating assembly, the signal output driver circuit being configured to transmit the signal from the signal generating assembly to a driver module configured to generate RF energy, the signal being configured to inhibit the driver module form generating RF energy when the amount of fluid within the fluid source reaches a predetermined threshold amount.

### Example 20

The apparatus of Example 19, the signal output driver circuit being configured to selectively couple with a cable, the cable being configured to selectively couple with the driver module.

### Example 21

An apparatus, comprising: (a) a body; (b) a fluid bag support coupling configured to support a fluid source; and (c) a signal generating assembly, comprising: (i) a power source, (ii) a load cell assembly attached to the body and the fluid bag support coupling, at least a portion of the load cell assembly being in electrical communication with the power source, the load cell assembly being configured to generate a signal in response to an amount of fluid within the fluid source, and (ii) a receiver assembly in electrical communication with the power source and the load cell assembly, the receiver assembly being configured to transmit the signal from the signal generating assembly to a working element configured to perform a task, the signal being configured to inhibit the working element from performing the task when the amount of fluid within the fluid source reaches a predetermined threshold amount.

### V. Miscellaneous

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) a fluid bag support coupling configured to support a fluid source;
(b) a signal generating assembly coupled to the fluid bag support coupling, the signal generating assembly being configured to generate a signal in response to an amount of fluid within the fluid source; and
(c) a signal output driver circuit in electrical communication with the signal generating assembly, the signal output driver circuit being configured to transmit the signal from the signal generating assembly to a working element configured to perform a task, the signal being configured to inhibit the working element from performing the task when the amount of fluid within the fluid source reaches a predetermined threshold amount.

2. The apparatus of claim 1, the signal generating assembly comprising a load cell assembly.

3. The apparatus of claim 1 or claim 2, the signal generating assembly comprising a power source.

4. The apparatus of claim 3, the signal generating assembly comprising a receiver in electrical communication with the load cell assembly and the power source.

5. The apparatus of claim 4, the apparatus comprising a digital display, the receiver being configured to transmit the signal to a digital display.

6. The apparatus of claim 4 or claim 5, further comprising an audible alarm, the receiver being configured to activate the audible alarm when the amount of fluid within the fluid source reaches the predetermined threshold amount.

7. The apparatus of any one of claims 1 to 6, further comprising a user input assembly configured to calibrate the signal generating assembly.

8. The apparatus of claim 7, the user input assembly further comprising a power button configured to activate the apparatus.

9. The apparatus of claim 7 or claim 8, the input assembly further comprising an alarm button configured to deactivate the audible alarm.

10. The apparatus of any one of claims 1 to 9, the signal generating assembly comprising a run dry sensor.

11. The apparatus of any one of claims 1 to 10, the working element comprising a pump in fluid communication with the fluid source, the pump being configured to pump fluid out of the fluid source.

12. The apparatus of any one of claims 1 to 11, the working element comprising a driver module configured to activate RF energy.

13. The apparatus of any one of claims 1 to 12, the signal generating assembly comprising a strain gauge.

14. The apparatus of claim 13, the signal generating assembly comprising an elastic body fixed to the strain gauge.

15. The apparatus of any one of claims 1 to 14, further comprising a body housing at least a portion of the signal generating assembly.

16. The apparatus of claim 11 when dependent upon claim 1, further comprising an audible alarm, the signal generating assembly being configured to activate the audible alarm when the fluid source reaches the predetermined threshold amount.

17. The apparatus of claim 12 when dependent upon claim 1 , the signal output driver circuit being configured to selectively couple with a cable, the cable being configured to selectively couple with the driver module.
